# EUROPEAN PATENT APPLICATION

(11) **EP 1 953 144 A1**
(43) Date of publication of application: **06.08.2008**
(21) Application number: 07101421.1
(22) Date of filing: 30.01.2007
(51) Int. Cl.: C07D 231/14, A61K 31/415, A61P 3/00

(54) **Novel polymorphic forms of N-piperidino-5-(4-chlorophenyl)-1-(2, 4-dichlorphenyl)-4-methyl-3-pyrazolecarboxamide**

(71) Applicant: SANDOZ AG, 4056 Basel (CH)
(72) Inventor: Ludescher, Johannes, 6252 Breitenbach (AT); Griesser, Ulrich J., 6094 Axams (AT); Langes, Christoph, 6020 Innsbruck (AT); Fucke, Katherina, 65812 Bad Soden (DE); Wieser, Josef, 6403 Polling (AT)
(74) Representative: Kröger, Bernd

(57) **Abstract**

The present invention relates to novel polymorphic forms of N-piperidino-5-(4-chlorophenyl)-1-(2, 4-dichlorphenyl)-4-methyl-3-pyrazolecarboxamide and to novel methods for their preparation. More particularly the invention relates to the preparation of a monohydrate of N-piperidino-5-(4-chlorophenyl)-1-(2, 4-dichlorphenyl)-4-methyl-3-pyrazolecarboxaniide rimonabant and to a novel anhydrous form of N-piperidino-5-(4-chlorophenyl)-1-(2, 4-dichlorphenyl)-4-methyl-3-pyrazolecarboxamide, and to pharmaceutical compositions thereof.

## Description

The present invention relates to novel polymorphic forms of N-piperidino-5-(4-chlorophenyl)-1-(2, 4-dichlorphenyl)-4-methyl-3-pyrazolecarboxamide and to novel methods for their preparation. More particularly the invention relates to the preparation of a monohydrate of N-piperidino-5-(4-chlorophenyl)-1-(2, 4-dichlorphenyl)-4-methyl-3-pyrazolecarboxamide rimonabant called HY1 and to a novel anhydrous form of N-piperidino-5-(4-chlorophenyl)-1-(2, 4-dichlorphenyl)-4-methyl-3-pyrazolecarboxamide, hereinafter called form III, and to pharmaceutical compositions thereof.

### N-piperidino-5-(4-chlorophenyl)-1-(2, 4-dichlorphenyl)-4-methyl-3-pyrazolecarboxamide of formula I

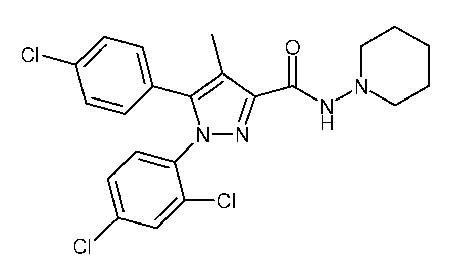

With the international nonproprietary name of rimonabant, is an antagonist of the CB1 cannaboid receptors, and was described e.g. in EP 0656354. The EP 0656354 also describes a process for the preparation of rimonabant yielding a form called form I. US2005/0043356 discloses the preparation of rimonabant in a form called form II.

Polymorphism is a phenomenon relating to the occurrence of different crystal forms for one molecule. There may be several different crystalline forms for the same molecule with distinct crystal structures and varying in physical properties like melting point, XRPD spectrum and IR-spectrum. These polymorphs are thus distinct solid forms which share the molecular formula of the compound from which the crystals are made up, however they may have distinct advantageous physical properties. These properties can have a direct effect on the ability to process and/or manufacture a final dosage form from the specific polymorphic form, for example improved flowability, as well as on the stability of the polymorphic form as such or the stability of it in a final dosage form, as well as its dissolution behavior and bioavailability.

There is thus a need for new crystalline forms of rimonabant and for processes for the production of such crystalline forms.

### Summary of the invention

The present inventors have now found two novel polymorphic forms of rimonabant which differ from each other and from the prior art forms in their stability, in their physical properties, in their spectral characteristics and in their methods of preparation.

Thus the present invention relates to a novel monohydrate of rimonabant called form HY1. HY 1 advantageously is stable upon exposure to high relative humidity and shows very advantageous flowability characteristics. For example, it does not form clumps during formulation.

Form HY 1 may, for example, be characterized by an XRPD spectrum comprising peaks at 2 Theta values of 7.2 ± 0.2, 9.3 ± 0.2, 10.5 ± 0.2, 13.5 ± 0.2, 17.0 ± 0.2, 17.8 ± 0.2 and 20.7 ± 0.2.

The present invention also relates to a novel anhydrous form of rimonabant called rimonabant form III. Form III has improved flowability properties at low relative humidity.

The present invention also relates to mixtures of these two novel poloymorphic forms.

The present invention also relates to pharmaceutical compositions comprising form HY1 or form III or mixtures thereof.

In another aspect the present invention discloses processes for the preparation of form HY1.

Furthermore, the present invention discloses a process for the preparation of form III. These two forms can be obtained by using particular crystallization conditions and/or drying conditions.

### Detailed description of the invention

The present inventors have surprisingly identified new polymorphic forms of Rimonabant which are stable upon storage. This property is important and proves advantageous for the desired use of Rimonabant in pharmaceutical formulations.

The invention therefore relates in a first embodiment to Crystalline rimonabant monohydrate.

Crystalline Rimonabant monohydrate may be characterized by having a water content of between 2.8% and 4.5% KF, in particular of from 3.4% to 4.1% KF. It may alternatively be characterized by displaying a loss of between 0.8 and 1.2 mole of water per mole of rimonabant when being heated from 20°C to 110°C.

In a preferred embodiment the invention relates to Crystalline rimonabant monohydrate with a PXRD (X-ray powder diffraction pattern) comprising peaks at 2 Theta angles of 7.2 ±0.2, 9.3 ±0.2, 10.5 ±0.2, 13.5 ±0.2, 17.0±0.2, 17.8±0.2, and 20.7±0.2, in particular of 7.22 ±0.1, 9.26 ±0.1, 10.45 ±0.1, 13.49 ±0.1, 17.05±0.1, 17.77±0.1, and 20.73±0.1.

In an alternative preferred embodiment the invention relates to Crystalline rimonabant monohydrate which shows, when analyzed by DSC (differential scanning calorimetry), a peak between 90°C and 105°C, preferably at about 98°C, followed by a further peak at between 105°C and 120°C, preferably at about 112°C.

In a very preferred embodiment, the invention relates to Crystalline rimonabant monohydrate with a PXRD comprising peaks at 2 Theta angles of 13.5 ±0.2°, and 17.76 ±0.2, and showing a peak in a DSC at between between 90°C and 105°C, in particular Crystalline rimonabant monohydrate with a PXRD spectrum comprising peaks at 2 Theta angles of 7.2 ±0.2, 9.3 ±0.2, 10.5 ±0.2, 13.5 ±0.2, 17.0±0.2, 17.8±0.2, and 20.7±0.2 and showing a peak in a DSC between 90°C and 120°C, preferably at about 98°C, followed by a further peak at between 105°C and 115°C, preferably at about 112°C.

Exemplary 2 theta values and intensities of XRPD peaks observed for rimonabant monohydrate form HY1 are presented in table 1

**Table 1**

| Rimonabant Hy1 | |
|---|---|
| High resolution XRPD | |
| Angle | rel. int |
| 2-Theta ° | % |
| 7.2 ± 0.2 | 14 |
| 9.3 ± 0.2 | 54 |
| 10.5 ± 0.2 | 31 |
| 13.5 ± 0.2 | 94 |
| 17.0 ± 0.2 | 67 |
| 17.8 ± 0.2 | 100 |
| 20.7 ± 0.2 | 39 |

The present invention also relates to processes for the preparation of Rimonabant monohydrate.

Crystalline rimonabant monohydrate form HY1 may prepared by providing a solution or suspension of rimonabant hydrochloride or rimonabant free base in an organic solvent and by then adding sufficient water to induce the crystallization of the monohydrate form HY1.

Alternatively the solvent of an organic aqueous suspension or solution of rimonabant hydrochloride or rimonabant base may be removed by conventional methods, e.g. evaporation or distillation whereupon the hydrate form HY1 crystallizes.

The term "sufficient amount of water" is to be understood as to have sufficient amount of water present in the solution or suspension as to induce crystallization of rimonabant hydrate form HY1 respectively as to have the rimonabant hydrochloride dissociate into rimonabant base, because rimonabant is only a relatively weak base.

Alternatively a base may be added to a solution or suspension of rimonabant hydrochloride , such as e.g. a alkali- or earth alkali- carbonate, a base selected from an alkali- or earth alkali- hydroxide or an organic base, such as an amine, a guanidine or amidine or a salt of a carboxylic acid transforming the rimonabant hydrochloride to rimonabant free base.

Suitable organic solvents are e.g. alcohols, e.g. C₁-C₆ alcohols, ketones, e.g. C₃-C₈ ketones, e.g. acetone, or amides, e.g. N, N-dimethylformamide or N, N-dimethylacetamide, sulfoxydes, e.g. DMSO or sulfones, e.g. sulfolane or nitriles, e.g. acetonitrile.

Crystalline rimonabant monohydrate form HY1 may, for example, also be prepared by dissolving the compound as free base or hydrochloride in a protic solvent such as methanol, and then precipitating it by adding water, or by evaporating the protic solvent from a suspension or solution of rimonabant base or rimonabant hydrochloride in a mixture of a protic solvent, such as methanol, and water, for example from a mixture of methanol and water.

The hydrate form HY may also be prepared by preparing a slurry of the compound as base or hydrochloride in a mixture of an organic solvent and water, e.g. in a mixture of a protic solvent and water, e.g. methanol and water, for a time suffucuent to induce crystallization of the novel hydrate.

The protic solvent may preferably be a C1-C6 alcohol, preferably methanol.

The invention further relates to the use of Rimonabant monohydrate for the production of a pharmaceutical composition. The invention further relates to a pharmaceutical composition comprising Rimonabant monohydrate, preferably in a pharmaceutically effective amount.

The present invention also relates to the novel crystalline anhydrous form III of rimonabant.

Crystalline Rimonabant form III may be characterized by having a water content of between 0.2 % and 0.8% KF, in particular of from 0.3% to 0.7% KF. It may alternatively be characterized by displaying a loss of between 0.1 and 0.3 mole of water per mole of rimonabant when being heated from 20°C to 110°C.

In a preferred embodiment the invention relates to Crystalline rimonabant form III with a PXRD (X-ray powder diffraction pattern) comprising peaks at 2 Theta angles of 9.3 ± 0.2, 10.2 ± 0.2, 15.0 ± 0.2, 16.1 ± 0.2, 16.9 ± 0.2, 21.1 1 ± 0.2 and 22. 8 ± 0.2.

In a very preferred embodiment, the invention relates to Crystalline rimonabant form III with a PXRD comprising peaks at 2 Theta angles of 16.1 ± 0.2, 21.1 ± 0.2 and 22. 8 ± 0.2, and having a water content of from 0.2% and 0.8% KF.

Results from an exemplary XRPD of form III are presented in table 2

**Table 2: characteristic angles 2 theta and relative intensities of rimonabant form III**

| Rimonabant form III | |
|---|---|
| Angle | rel Int |
| 2-Theta ° | % |
| 9.3 ± 0.2 | 40 |
| 10.2 ± 0.2 | 24 |
| 15.0 ± 0.2 | 38 |
| 16.1 ± 0.2 | 60 |
| 16.9 ± 0.2 | 38 |
| 21.1 ± 0.2 | 100 |
| 22.8 ± 0.2 | 70 |

The present invention also relates to a process for the production of form III of rimonabant. Form III may be prepared by drying the hydrate form HY1 of rimonabant under 'vigorous conditions' or by storing the hydrate form HY1 rimonabant at a relative humidity of less than e.g. about 20%, e.g. less than 10%, e.g. about 5% relative humidity. Water from the hydrate HY1 is lost and form III of rimonabant is formed. Vigorous drying may, for example, be effected by drying rimonabant hydrate form HY1 in vacuo in present of a strong desiccant, e.g. P2O5, optionally at elevated temperatures or in the presence of a strong vacuum, e.g. less than about 1 mbar.

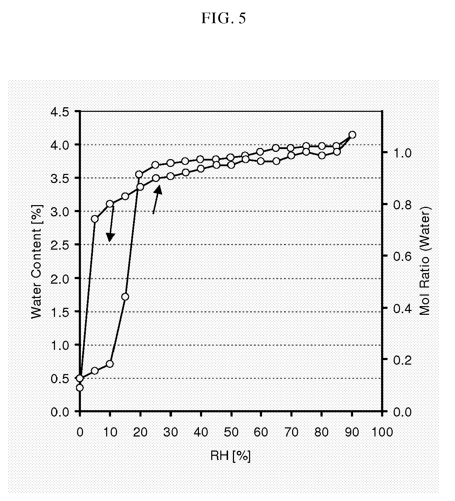

Crystalline form III of rimonabant may be converted to the crystalline hydrate HY1 of rimonabant by exposure of form III to a relative humidity of about 10% to 20 % relative humidity. The crystalline hydrate HY1 may be converted to crystalline form III of rimonabant by exposure of form HY1 to a relative humidity of about less than 10 % relative humidity, e.g. 10% to 5 % relative humidity as shown by the moisture sorption / desorption isotherm in figure 5.

The crystalline hydrate HY1 of rimonabant as well as the crystalline form III of rimonabant is stable. Furthermore the crystalline hydrate HY1 and the crystalline form III may be obtained in a specific manner by means of the methods of the invention. ; this constitutes an advantage for the industrial manufacture of the hydrate HY1 and form III of rimonabant.

Thus the crystalline hydrate HY1 of rimonabant and the crystalline form III of rimonabant or mixtures thereof with form crystalline form III of rimonabant are particularly suitable for the manufacture of pharmaceutical compositions containing as active ingredient, rimonabant in the form of monohydrate HY1 or in the form of form III or mixtures comprising these forms.

The pharmaceutical compositions of the present invention may be for oral, sublingual, subcutaneous, intramuscular, intraveneous, transdermal or local administration. Therein, the rimonabant in the form of monohydrate HY1 or in the form of form III or the mixtures comprising these forms can be administered optionally in combination with another active ingredient / other active ingredients, for example in single dose administration forms, and /or as a mixture with conventional vehicles. Appropriate single dose administration forms may comprise forms for the oral route, such as gelatin capsules, tablets, pills, powders, granules, and oral solutions or suspensions, for sublingual and buccal administration, aerosols, implants, forms for local (topical) administration, for transdermal administration, for subcutaneous administration, for intramuscular administration, for intravenous administration, for intranasal administration, or for intraocular administration administration.

In the pharmaceutical compositions of the present invention, the rimonabant in the form of monohydrate HY1 or in the form of form III or the mixtures comprising these forms are generally formulated as dosage units containing about 0.5 to 300 mg, advantageously from about 5 to about 60 mg, preferably from about 5 to about 40 mg per dosage unit, for daily administrations, once or several times a day.

Although the dosages are examples of average situations, there may be cases, depending on the weight and physical constitution of a subject to be treated, where higher or lower dosages are appropriate, such dosage forms are also part of the invention.

When a solid composition is prepared in form of tablets or gelatin capsules, a mixture of pharmaceutical excipients is added to the micronized or nonmicronized active ingredients, which mixture can be composed of diluents, e.g. for example lactose, mannitol,microcrystalline cellulose, starch or dicalcium phosphate, of binders , such as for example polyvinylpyrrolidone or hydroxypropylcellulose, or disintegrating agents, such as crosslinked polyvinylpyrrolidone or crosslinked carboxymethylcellulose, crosscarmellose sodium, or flow agents such as silica or talc, or of lubricants, such as magnesium stearate, stearic acid, glyceril tribehenate or sodium stearylfumarate.

Wetting agents or surfactants, such as sodium lauryl sulphate, polysorb 80 or poloxamer 188 can be added to the formulation.

The tablets may be prepared by various techniques, e.g. dry granulation wet granulation, hot melt or direct tabletting.

The tablets can be bare or sugar coated with various polymers or other appropriate materials.

The tablets can have a flash, delayed or sustained release by preparing polymeric matrices or by using specific polymers when forming the thin film.

A preferred composition is a dose unit of 20 mg in the form of tablets consisting of a core containing as the active ingredient rimonabant in the form of monohydrate HY1 or in the form of form III or the mixtures comprising these forms, starch, lactose monohydrate, povidon K30, sodiumdocecylsulphate, microcrystalline cellulose and magnesium stearate and a coating consisting of lactose monohydrate, hypromellose, titanium dioxide and macrogol 300.

The gelatin capsules may be soft or hard and may or not may be coated with a thin film, so as to have a flash, sustained or delayed activity, e.g. an enteric form. They can comprise not only a solid formulation formulated as above for tablets, but also liquids or semi liquids.

A preparation in the form of a syrup or elixir can comprise as the active ingredient rimonabant in the form of monohydrate HY1 or in the form of form III or the mixtures comprising these forms facultatively comprising a further active ingredient, in conjunction with a sweetener, preferably a calorie-free sweetener, methylparaben, and propylparaben, as antiseptic, as well as flavoring agent and an appropriate colorant.

The water-dispersible powders or granules can comprise as the active ingredient rimonabant in the form of monohydrate HY1 or in the form of form III or the mixtures comprising these forms, and facultatively a further active ingredient, as a mixture with dispersing agents, wetting agents or suspending agents such as polyvinylpyrrolidone or polyvidone as well as with sweeteners or taste coregents.

For parenteral, intranasal or intraocular administration, use is made of aqueous suspensions, isotonic saline solutions or sterile or injectable solutions which comprise, in addition to rimonabant in the form of monohydrate HY1 or in the form of form III or the mixtures comprising these forms, pharmacologically compatible dispersing agents and/or solubilizing agents, e.g. propylene glycol or butylenes acid and triglycol. Thus, to prepare an aqueous solution which can be injected by the intravenous route, use may be made of a co solvent, e.g. an alcohol, e.g. ethanol or a glycol, such as polyethylene glycol or propylene glycol and of a hydrophilic surfactant, such as polysorbate 80 or poloxamer 188. to prepare an oily solution which can be injected by the intramuscular route. For example, the active ingredient rimonabant in the form of monohydrate HY1 or in the form of form III or the mixtures comprising these forms may be dissolved with a triglyceride or a glyceryl ester.

For rectal administration recourse is made to suppositories which are prepared with binders which melt at the rectal temperature, e.g. polyethylene glycols.

For local administration use may be made of creams, ointments gels, eyewashes or sprays.

For transdermal administration use may be made of patches in multilaminar or reservoir form in which the active ingredient can be in alcoholic solution.

For administration by inhalation use may be made of an aerosol comprising e.g. sorbitan trioleate

And trichlorofluoromethane, dichlorofluoromethane, dichlorotetrafluoroethane, Freon substitutes or any other biologically compatible propellant gas. Use may also be made of a system comprising the active ingredient alone or in combination with an excipient in powder form.

The active ingredient rimonabant in the form of monohydrate HY1 or in the form of form III or the mixtures comprising these forms, facultatively in the presence of a further active ingredient, can also formulated in the form of microcapsules or microsphers, optionally with one or more vehicles or additives.

Use may be made of implants among the sustained release forms of use in the case of chronic treatment. These implants can be prepared in the form of an oily suspension or in the form of an oily suspension or in the form of a suspension of microspheres is an isotonic medium.

In the above described pharmaceutical formulations rimonabant in the form of monohydrate HY1 or in the form of form III or the mixtures comprising these forms, facultatively in the presence of a further active ingredient, can also be presented in form with a cyclodextrin e.g. α-, β-, or γ- cyclodextrin or 2-hydroxypropyl-ß-cyclodextrin or methyl-ß-cyclodextrin.

Preferably, rimonabant in crystalline hydrate form HY1 or rimonabant form III or mixtures thereof are administered by the oral route, as a single dose per day.

According to another aspect the invention also relates to a method for the treatment of appetency disorders, e.g. selected from the group consisting of the regulation of consumption desires, disorders associated with a substance, food behaviours, obsesity, obesity associated with non-insulin-dependent diabetes, any disease resulting in the patient becoming overweight, bulimia, drug abuse, drug dependency, the desire to consume non-essential food items and the spontaneous appetency for food items which usually brings pleasure, which comprises, and preferably consists of, administering a therapeutically effective quantity of rimonabant in the form of monohydrate HY1 or in the form of form III or the mixtures comprising these forms. Alternatively rimonabant in the form of the monohydrate or in the form of form III is to be administered in combination with a compound for regulating metabolic disorders, especially a ß3-adreneergic antagonist.

Rimonabant may also be administered in combination with a PPAR-antogonist for controlling appetites, in combination with bupropion to affect weight loss, in combinations with opoid antagonists affecting weight loss, in combination with sibutramine for the treatment of obesity, or combination with PDE 10-inhibitor for treating obesity.

Rimonabant may also be administered in combination with cannabinoid receptor modulators, as combination therapy with HMG CoA-reductase inhibitors, in combination with ezetimibe, in combination with D4/5-HT2A antagonists, in combination with lipase inhibitors, in combination with COX-2-inhibitors for the treatment of CNS damage or obesity, in combination with aldosterone receptor antagonists. A further medical use of rimonabant is in CB1-receptor related diseases in juvenile patients. Rimonabant may also be used in combination with C-aryl-glycides to treat diabetis, in combination with other therapeutic agents in diabetis therapy to treat diabetis, or in combination with KATP-channel openers. The invention therefore also relates to methods of treating the above-mentioned diseases with the above mentioned combinations, to the above-mentioned combinations of rimonabant in crystalline hydrate form HY1 or rimonabant form III with any one of the above-mentioned combination partners and to the use of rimonabant in crystalline hydrate form HY1 or rimonabant form III, in combination with any one of the above mentioned combination partners for the preparation of a medicament for the treatment of any one of the corresponding conditions or diseases mentioned above.

### Brief description of the drawings:

Fig 1 represents the high resolution XRPD of crystalline rimonabant hydrate form HY1
Fig 2 represents the DSC of crystalline rimonabant hydrate form HY1
Fig 3 represents the TGA of crystalline rimonabant hydrate form HY1
Fig 4 represents the XRPD of crystalline form III of rimonabant
Figure 5: moisture sorption / desorption of crystalline rimonabant hydrate form HY1 and crystalline rimonabant form III.

### Experimental

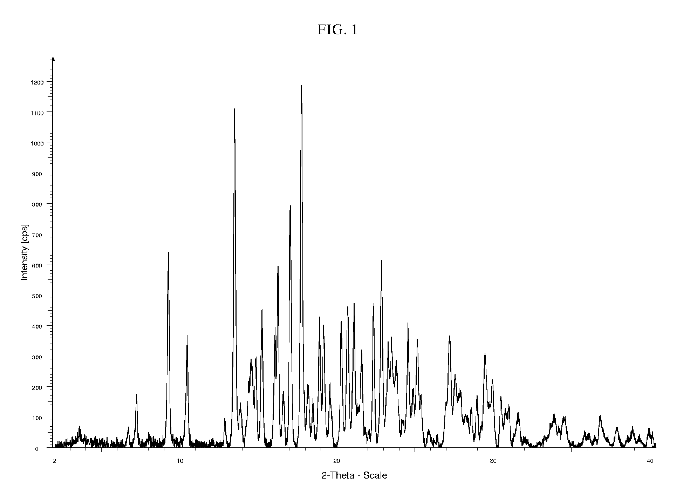

The X-ray powder diffraction pattern (XRPD) for the crystalline hydrate form HY1 ofrimonabant (figure 1) was taken on a STOE STADI MP powder diffractometer in transmission geometry with a 5° position sensitive detector using Cu Kα radiation, 6 mm axial beam height and 8 mm sample diameter. A diffraction angle of 2°-60° was measured with a total measuring time of 4 h (PSD step size 0.01°, 1s/step, step size of internal integration 0.01°).

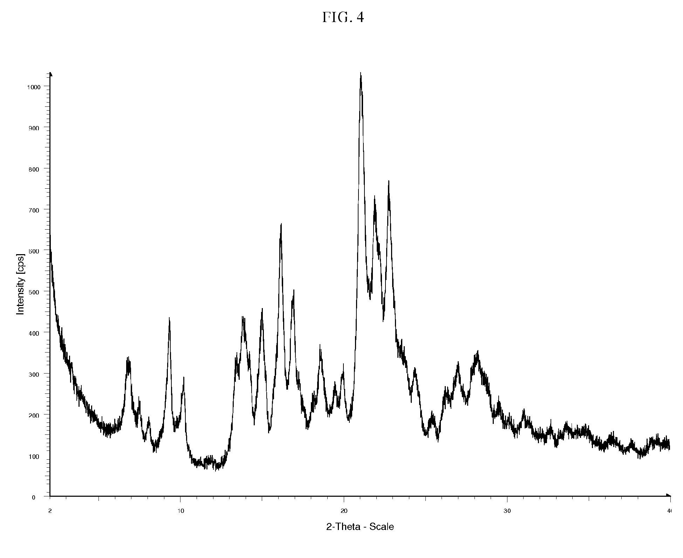

The X-ray powder diffraction pattern (XRPD) for the crystalline form III of rimonabant (figure 4) was obtained with a Siemens D-5000 diffractometer (Bruker-AXS, Karlsruhe, Germany) equipped with a theta/theta coupled goniometer, CuKα1 radiation source (wavelength 0,15406 nm), parallel beam optics (Goebel mirror, Bruker AXS, Karlsruhe, Germany), a 0.15° soller slit collimator and a scintillation counter. The pattern was recorded at a tube voltage of 40 kV and a tube current of 35 mA, applying a scan rate of 0.005° 2 theta s-1 in the angular range of 2° to 40° 2 theta. The sample was prepared on a glass sample holder.

### DSC of rimonabant hydrate form HY1

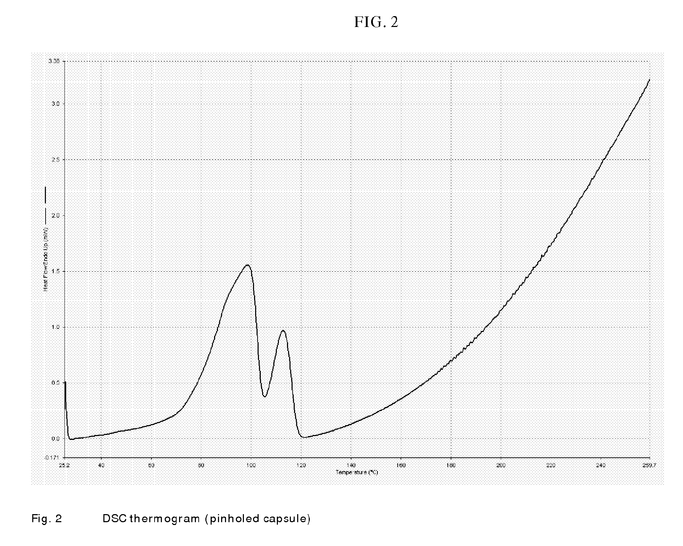

The DSC measurement was conducted using a DSC 7 (Perkin Elmer, Norwalk, Ct, USA), operated with the Pyris 2.0 software (figure 2). A sample of about 1 mg was accurately weighed (± 0.0005 mg) into an pinholed Al-Pan (30 µl) on a UM3 ultramicrobalance, (Mettler, Greifensee, CH), and scanned though the range of 25 to 260 °C at a heating rate of 10 K/min. Dry nitrogen was used as purge gas (purge: 20 ml min-1).

The DSC of rimonabant hydrate form HY1 shows the dehydration endotherm followed by the melting process in the range of 105°C to 120°C. The melting process was confirmed by hot stage microscope microscopy.

### TGA of rimonabant hydrate form HY1

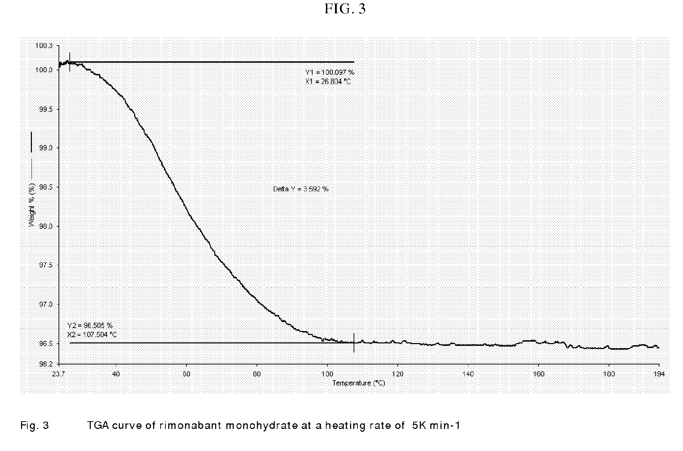

The TGA curve (figure 3) was recorded using a TGA 7 thermogravimetric system (Perkin-Elmer, Norwalk, Ct., USA) operated with the Pyris 2.0 software. Approximately 1 mg was accurately weighted (±0.0005 mg) into a 50 µl platin pan and subjected to non-isothermal TGA at a heating rate of 5 K/min in a temperature range from 25 to 200 °C. Nitrogen was used as a purge gas (balance purge: 50 ml/min, sample purge: 25 ml/min).

The sample analyzed by TGA was dried over silica gel for 12 hours to remove surface absorbed water but not the water incorporated in the crystal lattice. The TGA revealed a mass loss of 3.59 % by the experiment, thus representing a stoichiometric hydrate (theoretical water content 3.74%).

The moisture sorption isotherm (figure 5) was recorded with a SPS-11 moisture sorption analyzer (MD Messtechnik, Ulm, D). The measurement cycle was started at 30% relative humidity (RH), increased in 10% steps up to 90% RH, decreased in 10% steps down to 0% RH, increased in 1 step up to 30% RH and finally increased in 1 step up to 43% RH to determine subsequently the absolute water content by TGA analysis. The equilibrium condition for each step was set to a mass constancy of ±0.003 % over 35 min. The temperature was 25 ±0.1°C.

### Examples

The following examples describe the present invention in detail and are not to be construed to be in any way limiting to the scope of the claims.

### Example 1: Crystalline rimonabant form hydrate HY1

0.5 g of rimonabant hydrochloride is wetted with 2 drops methanol and approximately 1 ml of water. The paste is then stirred at room temperature for 12 hours and filtrated.

XRPD analysis demonstrated that the monohydrate form was obtained.

XRPD (high resolution) of Rimonabant monohydrate: most characteristic peaks are shown.

| Rimonabant monohydrate | | |
|---|---|---|
| High resolution -Transmission | | |
| Angle | d value | rel Int |
| 2-Theta ° | Angstrom | % |
| 7.21 | 12.251 | 14 |
| 9.26 | 9.547 | 54 |
| 10.45 | 8.459 | 31 |
| 13.49 | 6.559 | 94 |
| 17.04 | 5.199 | 67 |
| 17.76 | 4.991 | 100 |
| 20.70 | 4.287 | 39 |

### Example 2: crystalline rimonabant form hydrate HY1

0.2 g of rimonabant hydrochloride is dissolved in 0.5ml of methanol and the solution is quickly added to 5 ml water. The precipitate is amorphous and crystallizes within 12 hours at room temperature to the hydrate. The crystals are filtrated.

XRPD analysis demonstrated that monohydrate form HY1 was obtained.

### Example 3: crystalline rimonabant form hydrate HY1

0.31 g of rimonabant hydrochloride is suspended in 15 ml methanol/water 1:1 (Erlenmeyer flask, stirrer) and boiled uncovered to evaporate the methanol. The hydrate in the aqueous suspension is filtrated and dried over silica gel without applying vacuo overnight.

XRPD analysis demonstrated that monohydrate form HY1 was obtained.

### Example 4: crystalline rimonabant form hydrate HY1

0.2 g of rimonabant hydrochloride is suspended in 30 ml methanol/water 1:1 (100 ml flask) and the methanol is evaporated at 60 °C (water bath) and 520 mbar with the aid of a rotavapor. The solid is filtrated.

XRPD analysis demonstrated that monohydrate form HY1 was obtained.

### Example 5: crystalline rimonabant form hydrate HY1

0.23 g of rimonabant is suspended in a mixture of 0.7 ml of 2-propanole/water (1:1 v/v) and the mixture is stirred at ambient temperature for 6 hours. The product is then isolated by filtration.

XRPD analysis demonstrated that monohydrate form HY1 was obtained.

### Example 6: Rimonabant form III

0.2 g of rimonabant hydrate HY1 are dried over P205 for 12 hours and the dried product is analyzed by XRPD at a relative humidity of about 5% at ambient temperature. Form III was obtained.

| Rimonabant form I I I | | |
|---|---|---|
| Bragg Brentano | | |
| Angle | d value | rel Int |
| 2-Theta ° | Angstrom | % |
| 9.30 | 9.506 | 40 |
| 10.17 | 8.695 | 24 |
| 14.98 | 5.910 | 38 |
| 16.13 | 5.491 | 60 |
| 16.87 | 5.251 | 38 |
| 21.05 | 4.218 | 100 |
| 22.76 | 3.904 | 70 |

## Claims

1. Crystalline rimonabant hydrate.

2. Crystalline rimonabant hydrate of claim 1 having a water content of between 2.8% and 4.5% KF

3. Crystalline rimonabant hydrate of any one of claims 1 to 2, displaying a loss of between 0.8 and 1.2 mole of water per mole of rimonabant when being heated from 20°C to 110°C, when measured by TGA at a heating rate of 5 K/min.

4. Crystalline rimonabant hydrate of any one of claims 1 to 3 **characterized by** a XRPD (X-ray powder diffraction pattern) comprising peaks at 2 Theta angles of 7.2 ±0.2, 9.3 ±0.2, 10.5 ±0.2, 13.5 ±0.2, 17.0±0.2, 17.8±0.2, and 20.7±0.2, in particular of 7.22 ±0.1 , 9.26 ±0.1, 10.45 ±0.1, 13.49 ±0.1, 17.05±0.1, 17.77±0.1, and 20.73±0.1.

5. Crystalline anhydrous rimonabant, **characterized by** having a water content of between 0.2 % and 0.8% KF.

6. Crystalline anhydrous rimonabant, **characterized by** a XRPD (X-ray powder diffraction pattern) comprising peaks at 2 Theta angles of 9.3 ± 0.2, 10.2 ± 0.2, 15.0 ± 0.2, 16.1 ± 0.2, 16.9 ± 0.2, 21.1 ± 0.2 and 22. 8 ± 0.2.

7. A process for the preparation of crystalline rimonabant hydrate according to any one of claims 1 to 4, **characterized by** providing a solution of rimonabant base or hydrochloride in an organic solvent and inducing crystallization of said rimonabant hydrate by the addition of water.

8. A process for the preparation of crystalline rimonabant hydrate according to any one of claims 1 to 4, **characterized by** removing an organic solvent from a suspension or solution of rimonabant base or rimonabant hydrochloride in a mixture of an organic solvent and water.

9. A process for the preparation of crystalline rimonabant hydrate according to any one of claims 1 to 4, **characterized by** preparing a slurry of rimonabant base or rimonabant hydrochloride in a mixture of an organic solvent and water, and allowing for sufficient time to induce crystallization of said rimonabant hydrate.

10. A process for the preparation of crystalline rimonabant hydrate according to any one of claims 1 to 4, **characterized by** exposing crystalline anhydrous rimonabant according to any one of claims 5 to 6 to a relative humidity of greater than about 10% at ambient temperature

11. A pharmaceutical composition containing as active ingredient crystalline rimonabant hydrate according to any one of claims 1 to 4 in combination with at least one pharmaceutical excipient.

12. A pharmaceutical composition containing as active ingredient crystalline anhydrous rimonabant according to any one of claims 5 to 6 in combination with at least one pharmaceutical excipient.

13. A pharmaceutical composition containing as active ingredient a mixture of crystalline rimonabant hydrate according to any one of claims 1 to 4 and crystalline anhydrous rimonabant according to any one of claims 5 to 6, in combination with at least one pharmaceutical excipient

14. A method for treating diseases susceptible of administration of an effective amount of an antagonist of the CB1 cannabinoid receptor which method comprises administering effective amounts of crystalline rimonabant hydrate according to any one of claims 1 to 4 or crystalline anhydrous rimonabant according to any one of claims 5 to 6 or mixtures thereof, facultatively in combination with a further active pharmaceutical ingredient.

## Amended claims

### Amended claims in accordance with Rule 137(2) EPC.

**1.** Crystalline rimonabant hydrate.

**2.** Crystalline rimonabant hydrate of claim 1 having a water content of between 2.8% and 4.5% KF

**3.** Crystalline rimonabant hydrate of any one of claims 1 to 2, displaying a loss of between 0.8 and 1.2 mole of water per mole of rimonabant when being heated from 20°C to 110°C, when measured by TGA at a heating rate of 5 K/min.

**4.** Crystalline rimonabant hydrate of any one of claims 1 to 3 **characterized by** a XRPD (X-ray powder diffraction pattern) comprising peaks at 2 Theta angles of 7.2 ±0.2, 9.3 ±0.2, 10.5 ±0.2, 13.5 ±0.2, 17.0±0.2, 17.8±0.2, and 20.7±0.2, in particular of 7.22 ±0.1, 9.26 ±0.1, 10.45 ±0.1, 13.49 ±0.1, 17.05±0.1, 17.77±0.1, and 20.73±0.1.

**5.** A process for the preparation of crystalline rimonabant hydrate according to any one of claims 1 to 4, **characterized by** providing a solution of rimonabant base or hydrochloride in an organic solvent and inducing crystallization of said rimonabant hydrate by the addition of water.

**6.** A process for the preparation of crystalline rimonabant hydrate according to any one of claims 1 to 4, **characterized by** removing an organic solvent from a suspension or solution of rimonabant base or rimonabant hydrochloride in a mixture of an organic solvent and water.

**7.** A process for the preparation of crystalline rimonabant hydrate according to any one of claims 1 to 4, **characterized by** preparing a slurry of rimonabant base or rimonabant hydrochloride in a mixture of an organic solvent and water, and allowing for sufficient time to induce crystallization of said rimonabant hydrate.

**8.** A pharmaceutical composition containing as active ingredient crystalline rimonabant hydrate according to any one of claims 1 to 4 in combination with at least one pharmaceutical excipient.

**9.** A method for treating diseases susceptible of administration of an effective amount of an antagonist of the CB1 cannabinoid receptor which method comprises administering effective amounts of crystalline rimonabant hydrate according to any one of claims 1 to 4, facultatively in combination with a further active pharmaceutical ingredient.
